# EUROPEAN PATENT SPECIFICATION

(11) **EP 3 631 013 B1**
(45) Date of publication and mention of the grant of the patent: **11.09.2024**
(21) Application number: 18724158.3
(22) Date of filing: 30.04.2018
(51) Int. Cl.: C12Q 1/686, A24D 3/10

(54) **MARKED CELLULOSE ACETATE FIBRES, MANUFACTURING METHODS AND PRODUCTS COMPRISING SUCH FIBRES**
MARKIERTE CELLULOSEACETATFASERN, HERSTELLUNGSVERFAHREN UND PRODUKTE MIT SOLCHEN FASERN
FIBRES D'ACÉTATE DE CELLULOSE MARQUÉES, PROCÉDÉS DE FABRICATION ET PRODUITS CONTENANT DE TELLES FIBRES

(30) Priority: 31.05.2017 EP 17173747
(43) Date of publication of application: 08.04.2020
(73) Proprietor: Cerdia International GmbH, 4052 Basel (CH)
(72) Inventor: HOELTER, Dirk, 79312 Emmendingen (DE); LAPERSONNE, Philippe, 79285 Ebringen (DE)
(74) Representative: Meissner Bolte Partnerschaft mbB
(86) International application number: PCT/EP2018/061020
(87) International publication number: WO 2018/219567

(56) References cited:
- WO-A1-2015/200598
- WO-A1-2016/179220
- WO-A1-87/06383
- US-A1- 2009 272 392
- US-A1- 2011 207 125
- US-A1- 2015 018 538
- US-A1- 2015 377 841
- LODOVICI ET AL: "DNA solution^R in cigarette filters reduces polycyclic aromatic hydrocarbon (PAH) levels in mainstream tobacco smoke", FOOD AND CHEMICAL TOXICOLOGY, PERGAMON, GB, vol. 45, no. 9, 29 July 2007 (2007-07-29), pages 1752 - 1756, XP022176421, ISSN: 0278-6915, DOI: 10.1016/J.FCT.2007.03.007

## Description

The present invention concerns a process for the manufacture of marked cellulose acetate fibres as defined in claim 1, an apparatus for marking cellulose acetate fibres as defined in claim 6, a process for the detection of at least one information item on marked cellulose acetate fibres or on products comprising marked cellulose acetate fibres as defined in claim 7, a process for the manufacture of a tow bale comprising marked cellulose acetate fibres as defined in claim 10, a tow bale or tow as defined in claim 11, a filter as defined in claim 12 and a cigarette comprising marked cellulose acetate fibres as defined in claim 13.

Counterfeit products are causing damage to trade and governments, as illicit trading and counterfeiting harms originators by flushing the markets with illicit products and circumventing tax and customs regulations, thus harming governments aiming to enforce tax and customs laws. Attempts have been made to distinguish counterfeit products from original products by marking of products, making it possible to determine if a product is counterfeit or original. For example, US2016/0168781 A1 discloses a method of marking fibres, wherein fibres are being marked with a nucleic acid marker with the aim to determine whether the fibrous material is authentic or counterfeit. A similar method is known from WO 2015/200598 A1 describing labelling of cellulose acetate with specific fibers e.g.during the production process of cellulose acetate tows. From US 2011/207 125 A1, methods for labelling a product with a mixture of polynucleotides is known, wherefrom a major part of the polynucleotides "disguises" a minority of polynucleotides, which encode information. Such marking is used e.g. for tobacco. US 2015/018 538 A1 pertains to a method for marking articles with a nucleic acid marker to identify and authenticate the article's origin, wherein the article may comprise a textile material or may be an electronic product.

WO87/06383 A1 discloses hidden marker labels for products, wherein DNA is placed on cellulose acetate sheets. US 2009/272392 A1 is directed at cigarette filters, which are impregnated with double stranded DNA to capture pyrene residues from the cigarette smoke.

One of the products which are particularly prone to black market and counterfeiting are cigarettes, which are subject to tax and customs regulations in a large variety of countries. It is thus desirable to make cigarettes distinguishable with respect to original or counterfeit nature of the cigarette. WO2016/179220 A1 discloses, for example, marked cigarette filters, wherein the marking is achieved by etching, barcode or printing. Such markings can be subject to damage, caused by physical damage or biological disintegration or degradation of the filter, thus disabling the detecting mechanism. It is further possible that even these markings can be subject to imitation and counterfeiting.

There is thus still a need to provide marked cellulose acetate fibres, which are often comprised in cigarettes and upstream products thereof, which are marked in a manner which is more resistant to physical damage and biological disintegration of the cigarettes and upstream products thereof. It is further desirable to provide a marking for these products which makes it possible to trace the origin of such products. By providing information items, such as producer, date, place, batch, fibre characteristics and product line, a product comprising the marked cellulose acetate fibres can be traced by identifying the markings on the product to its origin. It is possible for involved parties, such as police and customs, to match the obtained information with production information from the product providers, which are able by identifying through warehouse and distribution information from where the product originates, where it was originally intended to be shipped and traded. This not only allows distinction between counterfeit and original products, but provides valuable information for the prosecution of criminal activities and responsible individuals and organizations.

The invention thus concerns a process for the manufacture of marked cellulose acetate fibres, which are comprised in cigarettes, comprising a step wherein a plurality of cellulose acetate fibres are provided; a step wherein a marker comprising at least one polynucleotide is deposited onto at least a portion of the cellulose acetate fibres, thereby marking the cellulose acetate fibres, wherein the at least one polynucleotide encodes information comprising at least one information item selected from the group consisting of producer, date, place, batch, fibre characteristics and product line. The invention further concerns an apparatus for marking cellulose acetate fibres with at least one polynucleotide as defined in claim 6; a process for the detection of at least one information item as defined in claim 7; a process for the manufacture of a tow bale comprising cellulose acetate fibres marked with at least one polynucleotide as defined in claim 10; tow, tow bale, filter and cigarette comprising such cellulose acetate fibres as defined in claim 11, 12 and 13, resp.

In the present invention, designations in singular are intended to include the plural; for example, "a fibre" is intended to denote also "more than one fibre" or "a plurality of fibres".

All aspects and embodiments of the present invention are combinable.

In the context of the present invention, the term "comprising" is intended to include the meaning of "consisting of".

The invention concerns a process for the manufacture of marked cellulose acetate fibres which are comprised in cigarettes, comprising:
- a step wherein a plurality of cellulose acetate fibres are provided; a step wherein a marker comprising at least one polynucleotide is deposited onto at least a portion of the cellulose acetate fibres, thereby marking the cellulose acetate fibres, wherein the at least one polynucleotide encodes information comprising at least one information item selected from the group consisting of producer, date, place, batch, fibre characteristics and product line.

The term "polynucleotide" intends to denote polymers comprising a plurality of nucleotides or nucleotide analogues. According to the present invention, "polynucleotide" denotes, for example, fragments of DNA or RNA, in particular artificial fragments of DNA or RNA. The length of the polynucleotide often is equal to or greater than 6 nucleotides, preferably equal to or greater than 12 nucleotides, and even more preferably equal to or greater than 15 nucleotides. The length of the polynucleotide often is equal to or less than 200 nucleotides, preferably equal to or less than 100 nucleotides, and even more preferably equal to or less than 50 nucleotides. Polynucleotides with a nucleotide length of from 6 to 200 nucleotides are also denoted as oligonucleotide. In one aspect of the present invention, the length of the polynucleotide can also exceed 200, such as 300, 400 or even more than 500 nucleotides. Lengths of up to 2000 nucleotides can be suitable. Preferably, the at least one polynucleotide comprises from 6 to 200 nucleotides. The nucleotides present in the polynucleotide are the naturally occurring nucleotides, such as adenine, cytosine, guanine, thymine and uracil, any of their derivatives, such as 5-fluorocytosine, 5-fluorouracil, 5-methylcytosine, 5-hydroxymethylcytosine, dideoxynucleotides, cordycepin and other artificial nucleotides and derivatives of nucleotides.

The polynucleotide according to the present invention can be a polynucleotide with a natural nucleotide sequence, such as plant or animal polynucleotide, for example obtained by digestion of plant or animal RNA or DNA, or, which is preferred, a non-natural polynucleotide with a predefined non-natural nucleotide sequence. The preferred non-natural polynucleotides can be obtained through methods skilled in the art, for example by applying the phosphoramidite method.

The at least one polynucleotide marker deposited on the cellulose acetate fibres preferably not only makes it possible to distinguish counterfeit from original products comprising such fibres. Such encoded information items generally can comprise any information suitable to detect the origin of the marked cellulose acetate fibres or products comprising these fibres. The information is encoded by the specific nucleotide sequence present in the at least one polynucleotide. Suitably, the sequence is confidential and specific for certain producers, dates, places, batches, fibre characteristics and product lines. Due to the high variability of the sequence and simultaneous high specificity of the sequence as defined by its originator, it is difficult if not possible to imitate these markers. A further advantage is that it is virtually impossible to remove the markers from the products, as it is known that by assaying techniques such as PCR even small amounts of marker suffice to allow detection. The marker generally is resistant against usual physical and biological stress to which the products comprising the marked cellulose acetate fibres can be exposed, and, due to its specificity, can be well distinguished from any artefacts, such as DNA from a different source.

The step of providing a plurality of cellulose acetate fibres can comprise the mere provision of cellulose acetate fibres which have been obtained from a commercial source. The manufacturing process can also comprise the following steps: (a) spinning cellulose acetate fibres from a dope comprising a solvent and cellulose acetate; (b) forming tow from the cellulose acetate fibres; (c) crimping the tow; (d) drying the tow; wherein the at least one polynucleotide is deposited onto at least a portion of the cellulose acetate fibres between or during either of steps (a) to (d). The general concept of steps (a) to (d) are known from e.g. P. Rustemeyer, Macromol. Symp. 2004, 208, 267-291. The deposition of the at least one polynucleotide can be effected on sections of the tow, or over the full length of the tow. The at least one polynucleotide preferably is applied as at least one aqueous or at least one alcoholic solution. In one aspect, the at least one polynucleotide is activated, for example alkaline activated, at deposition. Suitably, the deposition of the at least one polynucleotide is performed by metering a defined amount of at least one polynucleotide onto at least a portion of the cellulose acetate fibres or tow comprising cellulose acetate fibres. Alternatively, the cellulose acetate fibres can be dipped into at least one solution of the polynucleotide. In one aspect, the at least one polynucleotide is applied consecutively as two or more solutions. The at least one oligonucleotide can also be formulated into at least one solid formulation, such as a tablet. The solid formulation can comprise at least one auxiliary ingredient as known from, for example, the pharmaceutical or food industry, such as a binder or a coating. The solid formulation is then contacted with the at least one solvent, such as the aqueous or alcoholic solvent, prior to application to the cellulose acetate fibres.

According to the present invention, the deposition of the at least one polynucleotide on the cellulose acetate fibres or the tow comprising the cellulose acetate fibres can be effected between or during either of steps (a) to (d). In one aspect, the at least one polynucleotide can be present in the dope spun in step (a), or in the spinning auxiliary, for example spinning oil, employed in step (a). In a preferred aspect, the at least one polynucleotide is deposited onto at least a portion of the cellulose acetate fibres between steps (c) and (d). This has the advantage that any contamination of surroundings, fibres or tow can be minimized, and the at least one polynucleotide can be easily replaced for the next production batch with another polynucleotide. The risk of creating artefacts is minimized by this procedure. It has been shown that the deposition of the at least one polynucleotide before the drying step has no disadvantage with respect to stability or reliability of the marker.

The invention concerns further an apparatus for marking cellulose acetate fibres, the apparatus comprising:
- a transport system adapted to transport the cellulose acetate fibres comprised in cigarettes and obtainable by the above described method in a direction of a polynucleotide marker delivery apparatus positioned at a location along the transport system;
wherein the polynucleotide delivery apparatus comprises one or more outlets, adapted to deposit at least one solution comprising polynucleotide marker through the one or more outlets onto at least a portion of the fibres; and thereby marking the fibres.

Preferably, the apparatus has a metering device, making it possible to dose the exact amount per unit cellulose acetate fibre or tow or per time. Suitable transport systems adapted to transport the cellulose acetate fibres are known from the general concept of manufacturing cellulose acetate fibres and tows comprising such fibres, such as processes comprising spinning, crimping and drying steps.

Another object of the present invention is a process for the detection of at least one information item selected from the group consisting of producer, date, place, batch, fibre characteristics and product line comprised in the nucleotide sequence of the at least one polynucleotide on cellulose acetate fibres which are comprised in cigarettes and upstream products thereof marked with at least one polynucleotide comprising:
- obtaining a sample of the cellulose acetate fibres marked with at least one polynucleotide and
- assaying the sample of cellulose acetate fibres marked with at least one polynucleotide to identify the nucleotide sequence comprised in the at least one polynucleotide; and
- thereby detecting of the at least one information item selected from the group consisting of producer, date, place, batch, fibre characteristics and product line comprised in the nucleotide sequence of the at least one polynucleotide.

Products comprising cellulose acetate fibres marked with at least one polynucleotide can be, for example, cellulose acetate tow, filters comprising the cellulose acetate tow, tow bales comprising cellulose acetate tow and cigarettes comprising the filters comprising cellulose acetate fibres. The process for the detection of at least one information item can further comprise the step of extracting the at least one polynucleotide from the sample of cellulose acetate fibres marked with at least one polynucleotide or the products comprising cellulose acetate fibres marked with at least one polynucleotide, by suitable extraction methods such as treatment with an aqueous buffer.

A process for the detection of at least one information item selected from the group consisting of producer, date, place, batch, fibre characteristics and product line comprised in the nucleotide sequence of the at least one polynucleotide on cellulose acetate fibres which are comprised in cigarettes and upstream products thereof marked with at least one polynucleotide, further comprises: providing a plurality of cellulose acetate fibres; depositing at least one polynucleotide marker onto at least a portion of the cellulose acetate fibres; thereby producing marked cellulose acetate fibres.; The process may optionally comprise manufacturing a product comprising cellulose acetate fibres marked with at least one polynucleotide, for example manufacturing of filters from filter tow comprising cellulose acetate fibres marked with at least one polynucleotide. The assaying of the sample of cellulose acetate fibres marked with at least one polynucleotide or the products comprising cellulose acetate fibres marked with at least one polynucleotide to identify the nucleotide sequence comprised in the at least one polynucleotide preferably comprises a PCR technique.

The invention also concerns a process for the manufacture of a tow bale comprising cellulose acetate fibres as described above marked with at least one polynucleotide marker, which comprises the steps of (a) spinning cellulose acetate fibres from a dope comprising a solvent and cellulose acetate; (b) forming tow from the cellulose acetate fibres; (c) crimping the tow; (d) drying the tow; wherein the at least one polynucleotide is deposited onto at least a portion of the cellulose acetate fibres between or during either of steps (a) to (d), and which further comprises the steps of (e) baling the tow and (f) packaging the tow bale. It is preferred that the deposition of the at least one polynucleotide marker is performed between steps (c) and (d). The steps for (e) baling the tow and (f) packaging the tow bale are known, for example, from WO2016/174162 A1.

Another object of the present invention concerns cellulose acetate fibres marked with at least one polynucleotide marker. Such fibres are obtainable, for example, by the processes according to the present invention for concerning the manufacture of marked cellulose acetate fibres.

Yet another object of the present invention concerns tow or tow bale comprising cellulose acetate fibres marked with at least one polynucleotide marker according to the present invention.

The invention also concerns a filter comprising cellulose acetate fibres marked with at least one polynucleotide marker according to the present invention. The filter is a filter for gas, aerosol or liquid, preferably a filter for a gas or aerosol, and most preferably a cigarette filter filtering cigarette smoke. The manufacture of cigarette filters from tow comprising cellulose acetate fibres is generally known, for example from P. Rustemeyer, "Filter Rods and Cellulose Acetate - Life-Cycle from the Tree to the Consumer", 1998, 325023650, BATCo US DOJ v Philipp Morris, which can be obtained through
http)://legacy.library.ucsf.edu/tid/iun71a99pdf.

Another object of the present invention is a cigarette comprising cellulose acetate fibres marked with at least one polynucleotide marker, preferably comprising the filter according to the present invention.

Should the disclosure of any patents, patent applications, and publications which are mentioned herein conflict with the description of the present application to the extent that it may render a term unclear, the present description shall take precedence.

The following examples are intended to further explain the invention without limiting it.

### Example 1

A 12-mer oligonucleotide, encoding producer, product line and date was diluted in aqueous solution to a concentration of 2 µg/m³.

This oligomer solution was applied to the cellulose acetate fibres corresponding to about 0.3 ng oligonucleotide/kg cellulose acetate fibres, and the cellulose acetate fibres were dried.

A plurality of samples of the dried cellulose acetate fibres with a weight of 100 mg each were tested by routine DNA detection methods to provide forensic authentication. The oligonucleotide was detected after PCR amplification in every analysed sample, and the encoded information matched with the originating production batch. > 50 % of the oligonucleotide amount applied was detected.

## Claims

1. A process for the manufacture of marked cellulose acetate fibres, which are comprised in cigarettes, comprising:
- a step wherein a plurality of cellulose acetate fibres are provided;
- a step wherein a marker comprising at least one polynucleotide is deposited onto at least a portion of the cellulose acetate fibres, thereby marking the cellulose acetate fibres,
wherein the at least one polynucleotide encodes information comprising at least one information item selected from the group consisting of producer, date, place, batch, fibre characteristics and product line.

2. The process according to claim 1,
wherein the at least one polynucleotide comprises from 6 to 200 nucleotides.

3. The process according to claim 1 or 2,
wherein the at least one polynucleotide has a non-natural nucleotide sequence.

4. The process according to anyone of claims 1 to 3,
wherein the manufacturing process comprises:
(a) spinning cellulose acetate fibres from a dope comprising a solvent and cellulose acetate; (b) forming tow from the cellulose acetate fibres; (c) crimping the tow; (d) drying the tow; wherein the at least one polynucleotide is deposited onto at least a portion of the cellulose acetate fibres between or during either of steps (a) to (d).

5. The process according to anyone of claims 1 to 4,
wherein the at least one polynucleotide is deposited onto at least a portion of the cellulose acetate fibres between steps (c) and (d).

6. An apparatus for marking cellulose acetate fibres comprised in cigarettes and obtainable by the method according to one of the preceding claims, the apparatus comprising:
- a transport system adapted to transport the cellulose acetate fibres in a direction of a polynucleotide marker delivery apparatus positioned at a location along the transport system;
wherein the polynucleotide delivery apparatus comprises one or more outlets, adapted to deposit at least one solution comprising polynucleotide marker through the one or more outlets onto at least a portion of the fibres; and thereby marking the fibres.

7. A process for the detection of at least one information item selected from the group consisting of producer, date, place, batch, fibre characteristics and product line comprised in the nucleotide sequence of the at least one polynucleotide on cellulose acetate fibres which are comprised in cigarettes and upstream products thereof marked with at least one polynucleotide, comprising:
- obtaining a sample of the cellulose acetate fibres marked with at least one polynucleotide and
- assaying the sample of cellulose acetate fibres marked with at least one polynucleotide to identify the nucleotide sequence comprised in the at least one polynucleotide; and
- thereby detecting of the at least one information item selected from the group consisting of producer, date, place, batch, fibre characteristics and product line comprised in the nucleotide sequence of the at least one polynucleotide.

8. A process for the detection of at least one information item selected from the group consisting of producer, date, place, batch, fibre characteristics and product line comprised in the nucleotide sequence of the at least one polynucleotide on cellulose acetate fibres which are comprised in cigarettes and upstream products thereof marked with at least one polynucleotide, comprising:
- providing a plurality of cellulose acetate fibres;
- depositing at least one polynucleotide marker onto at least a portion of the cellulose acetate fibres;
- thereby producing marked cellulose acetate fibres;
- and further comprising the process according to claim 7.

9. The process according to claim 7 or 8,
wherein the assaying of the sample of cellulose acetate fibres marked with at least one polynucleotide or the products comprising cellulose acetate fibres marked with at least one polynucleotide to identify the nucleotide sequence comprised in the at least one polynucleotide comprises a PCR technique.

10. A process for the manufacture of a tow bale comprising cellulose acetate fibres which are comprised in cigarettes and upstream products thereof and are obtained by the process of claim 1 marked with at least one polynucleotide marker, which comprises the process according to claim 4 or 5, and which further comprises the steps of (e) baling the tow and (f) packaging the tow bale.

11. Tow or tow bale manufactured by the process of claim 10 comprising cellulose acetate fibres with at least one polynucleotide marker, wherein the at least one polynucleotide encodes information comprising at least one information item selected from the group consisting of producer, date, place, batch, fibre characteristics and product line.

12. Filter comprising cellulose acetate fibres comprised in cigarettes and manufactured by the process of claim 1 marked with at least one polynucleotide marker, preferably comprising the tow according to claim 11.

13. Cigarette comprising cellulose acetate fibres marked with at least one polynucleotide marker and manufactured by the process according to one of claims 1 to 5, preferably comprising the filter according to claim 12.

## Patentansprüche

1. Verfahren zur Herstellung von markierten Celluloseacetatfasern, die in Zigaretten enthalten sind, welches Folgendes aufweist:
- einen Schritt, bei dem eine Vielzahl von Zelluloseacetatfasern bereitgestellt wird;
- einen Schritt, bei dem ein Marker, der mindestens ein Polynukleotid aufweist, auf mindestens einen Teil der Celluloseacetatfasern aufgebracht wird, wodurch die Celluloseacetatfasern markiert werden,
wobei das mindestens eine Polynukleotid Informationen kodiert, die mindestens eine Information aufweisen, die aus der Gruppe ausgewählt ist, die aus Hersteller, Datum, Ort, Charge, Fasereigenschaften und Produktlinie besteht.

2. Verfahren nach Anspruch 1,
wobei das mindestens eine Polynukleotid 6 bis 200 Nukleotide aufweist.

3. Verfahren nach Anspruch 1 oder 2,
wobei das mindestens eine Polynukleotid eine nicht-natürliche Nukleotidsequenz aufweist.

4. Verfahren nach einem der Ansprüche 1 bis 3,
wobei das Herstellungsverfahren Folgendes aufweist:
(a) Spinnen von Celluloseacetatfasern aus einer Spinnlösung, die ein Lösungsmittel und Celluloseacetat aufweist; (b) Bilden von Tow aus den Celluloseacetatfasern; (c) Kräuseln des Tows; (d) Trocknen des Tows; wobei das mindestens eine Polynucleotid zwischen oder während eines der Schritte (a) bis (d) auf mindestens einen Teil der Celluloseacetatfasern aufgebracht wird.

5. Verfahren nach einem der Ansprüche 1 bis 4,
wobei das mindestens eine Polynukleotid auf mindestens einen Teil der Celluloseacetatfasern zwischen den Schritten (c) und (d) aufgebracht wird.

6. Vorrichtung zur Markierung von Zelluloseacetatfasern, die in Zigaretten enthalten sind und durch das Verfahren nach einem der vorhergehenden Ansprüche erhalten werden können, wobei die Vorrichtung Folgendes aufweist:
- ein Transportsystem, das dazu geeignet ist, die Zelluloseacetatfasern in Richtung einer Polynukleotidmarker-Abgabevorrichtung zu transportieren, die an einer Stelle entlang des Transportsystems angeordnet ist;
wobei die Polynukleotid-Abgabevorrichtung einen oder mehrere Auslässe aufweist, die so angepasst sind, dass sie mindestens eine Lösung, die einen Polynukleotidmarker aufweist, durch den einen oder die mehreren Auslässe auf mindestens einen Teil der Fasern auftragen und dadurch die Fasern markieren.

7. Verfahren zum Erfassen mindestens einer Information, die ausgewählt ist aus der Gruppe bestehend aus Hersteller, Datum, Ort, Charge, Fasereigenschaften und Produktlinie, die in der Nukleotidsequenz des mindestens einen Polynukleotids auf Zelluloseacetatfasern enthalten ist, die in Zigaretten und Vorprodukten davon enthalten sind, die mit mindestens einem Polynukleotid markiert sind, welches Folgendes aufweist:
- Gewinnen einer Probe der mit mindestens einem Polynukleotid markierten Zelluloseacetatfasern und
- Analysieren der Probe von Zelluloseacetatfasern, die mit mindestens einem Polynukleotid markiert sind, um die Nukleotidsequenz zu identifizieren, die in dem mindestens einen Polynukleotid enthalten ist; und
- dadurch Erfassen der mindestens einen Information, die ausgewählt ist aus der Gruppe bestehend aus Hersteller, Datum, Ort, Charge, Fasereigenschaften und Produktlinie, die in der Nukleotidsequenz des mindestens einen Polynukleotids enthalten ist.

8. Verfahren zum Erfassen mindestens einer Information, die ausgewählt ist aus der Gruppe bestehend aus Hersteller, Datum, Ort, Charge, Fasereigenschaften und Produktlinie, die in der Nukleotidsequenz des mindestens einen Polynukleotids auf Zelluloseacetatfasern enthalten ist, die in Zigaretten und Vorprodukten davon enthalten sind, die mit mindestens einem Polynukleotid markiert sind, welches Folgendes aufweist:
- Bereitstellen einer Vielzahl von Zelluloseacetatfasern;
- Aufbringen mindestens eines Polynukleotidmarkers auf mindestens einen Teil der Celluloseacetatfasern;
- wodurch markierte Zelluloseacetatfasern hergestellt werden;
- und ferner das Verfahren nach Anspruch 7 aufweisend.

9. Verfahren nach Anspruch 7 oder 8,
wobei das Analysieren der Probe von Celluloseacetatfasern, die mit mindestens einem Polynukleotid markiert sind, oder der Produkte, die mit mindestens einem Polynukleotid markierte Celluloseacetatfasern aufweisen, zur Identifizierung der Nukleotidsequenz, die in dem mindestens einen Polynukleotid enthalten ist, eine PCR-Technik aufweist.

10. Verfahren zur Herstellung eines Tow-Ballens, der Celluloseacetatfasern aufweist, die in Zigaretten und Vorprodukten davon enthalten sind und durch das Verfahren nach Anspruch 1 erhalten werden und die mit mindestens einem Polynucleotidmarker markiert sind, welches das Verfahren nach Anspruch 4 oder 5 aufweist, und welches ferner die Schritte (e) Ballenbildung des Tows und (f) Verpackung des Tow-Ballens aufweist.

11. Tow oder Tow-Ballen, der nach dem Verfahren des Anspruchs 10 hergestellt ist, welches/welcher Celluloseacetatfasern mit mindestens einem Polynucleotidmarker aufweist, wobei das mindestens eine Polynucleotid Informationen kodiert, die mindestens eine Information aufweisen, die aus der Gruppe bestehend aus Hersteller, Datum, Ort, Charge, Fasereigenschaften und Produktlinie ausgewählt ist.

12. Filter, der Celluloseacetatfasern aufweist, die in Zigaretten enthalten sind und durch das Verfahren des Anspruchs 1 hergestellt wurden, die mit mindestens einem Polynukleotidmarker markiert sind, und der vorzugsweise das Tow nach Anspruch 11 aufweist.

13. Zigarette, welche Zelluloseacetatfasern aufweist, die mit mindestens einem Polynucleotidmarker markiert sind, und durch das Verfahren nach einem der Ansprüche 1 bis 5, hergestellt sind, und vorzugsweise den Filter nach Anspruch 12 aufweist.

## Revendications

1. Procédé de fabrication de fibres d'acétate de cellulose marquées, qui sont comprises dans des cigarettes, comprenant:
- une étape dans laquelle une pluralité de fibres d'acétate de cellulose sont fournies;
- une étape dans laquelle un marqueur comprenant au moins un polynucléotide est déposé jusque sur au moins une portion des fibres d'acétate de cellulose, marquant ainsi les fibres d'acétate de cellulose,
dans lequel ledit au moins un polynucléotide encode des informations comprenant au moins un élément d'informations sélectionné parmi le groupe constitué de:
producteur, date, lieu, lot, caractéristiques de fibres et ligne de produit.

2. Procédé selon la revendication 1,
dans lequel ledit au moins un polynucléotide comprend de 6 à 200 nucléotides.

3. Procédé selon la revendication 1 ou 2,
dans lequel ledit au moins un polynucléotide a une séquence nucléotidique non naturelle.

4. Procédé selon l'une quelconque des revendications 1 à 3,
dans lequel le procédé de fabrication comprend les étapes consistant à:
- (a) filer des fibres d'acétate de cellulose à partir d'un dope comprenant un solvant et un acétate de cellulose;
- (b) former une étoupe à partir des fibres d'acétate de cellulose;
- (c) crêper l'étoupe;
- (d) sécher l'étoupe; dans lequel ledit au moins un polynucléotide est déposé jusque sur au moins une portion des fibres d'acétate de cellulose entre ou pendant l'une ou l'autre des étapes (a) à (d).

5. Procédé selon l'une quelconque des revendications 1 à 4,
dans lequel ledit au moins un polynucléotide est déposé jusque sur au moins une portion des fibres d'acétate de cellulose entre les étapes (c) et (d).

6. Appareil destiné à marquer des fibres d'acétate de cellulose comprises dans des cigarettes et pouvant être obtenues via le procédé selon l'une des revendications précédentes, l'appareil comprenant:
- un système de transport adapté pour transporter les fibres d'acétate de cellulose dans une direction d'un appareil de distribution de marqueur polynucléotidique positionné à un emplacement le long du système de transport;
dans lequel l'appareil de distribution de polynucléotide comprend une ou plusieurs sorties, adaptées pour déposer au moins une solution comprenant un marqueur polynucléotidique à travers les dites un ou plusieurs sorties jusque sur au moins une portion des fibres; et marquant ainsi les fibres.

7. Procédé de détection d'au moins un élément d'informations sélectionné parmi le groupe constitué de:
producteur, date, lieu, lot, caractéristiques de fibres et ligne de produit, comprenant la séquence nucléotidique dudit au moins un polynucléotide sur des fibres d'acétate de celluloses qui sont comprises dans des cigarettes et des produits en amont de celles-ci, marquées avec au moins un polynucléotide, comprenant les étapes consistant à:
- obtenir un échantillon des fibres d'acétate de cellulose marquées avec au moins un polynucléotide, et
- analyser l'échantillon de fibres d'acétate de cellulose marquées avec au moins un polynucléotide pour identifier la séquence nucléotidique comprise dans ledit au moins un polynucléotide; et
- détecter ainsi ledit au moins un élément d'informations sélectionné parmi le groupe constitué de:
producteur, date, lieu, lot, caractéristiques de fibres et ligne de produit, compris dans la séquence nucléotidique dudit au moins un polynucléotide.

8. Procédé de détection dudit au moins un élément d'informations sélectionné parmi le groupe constitué de:
producteur, date, lieu, lot, caractéristiques de fibres et ligne de produit, compris dans la séquence nucléotidique dudit au moins un polynucléotide sur des fibres d'acétate de celluloses qui sont comprises dans des cigarettes et des produits en amont de celles-ci, marquées avec au moins un polynucléotide, comprenant les étapes consistant à:
- fournir une pluralité de fibres d'acétate de cellulose;
- déposer au moins un marqueur polynucléotidique jusque sur au moins une portion des fibres d'acétate de cellulose;
- produire ainsi des fibres d'acétate de cellulose marquées;
- et comprenant en outre le procédé selon la revendication 7.

9. Procédé selon la revendication 7 ou 8,
dans lequel l'étape consistant à analyser l'échantillon de fibres d'acétate de cellulose marquées avec au moins un polynucléotide ou des produits comprenant des fibres d'acétate de cellulose marquées avec au moins un polynucléotide pour identifier la séquence nucléotidique comprise dans ledit au moins un polynucléotide comprend une technique PCR.

10. Procédé de fabrication d'une balle d'étoupe comprenant des fibres d'acétate de celluloses qui sont comprises dans des cigarettes et des produits en amont de celles-ci et qui sont obtenues via le procédé selon la revendication 1, marquées avec au moins un marqueur polynucléotidique, qui comprend le procédé selon la revendication 4 ou 5, et qui comprend en outre les étapes consistant à (e) mettre l'étoupe en balle et (f) conditionner la balle d'étoupe.

11. Étoupe ou balle d'étoupe fabriquée via le procédé selon la revendication 10, comprenant des fibres d'acétate de cellulose avec au moins un marqueur polynucléotidique,
dans laquelle ledit au moins un polynucléotide encode des informations comprenant au moins un élément d'informations sélectionné parmi le groupe constitué de: producteur, date, lieu, lot, caractéristiques de fibres et ligne de produit.

12. Filtre comprenant des fibres d'acétate de cellulose comprises dans des cigarettes et fabriquée via le procédé selon la revendication 1, marquées avec au moins marqueur polynucléotidique, de préférence comprenant l'étoupe selon la revendication 11.

13. Cigarette comprenant des fibres d'acétate de cellulose marquées avec au moins un marqueur polynucléotidique et fabriquées via le procédé selon l'une des revendications 1 à 5, comprenant de préférence le filtre selon la revendication 12.
